# EUROPEAN PATENT APPLICATION

(11) **EP 4 620 984 A1**
(43) Date of publication of application: **24.09.2025**
(21) Application number: 23891667.0
(22) Date of filing: 17.11.2023
(51) Int. Cl.: C08F 20/14, C08F 2/44, C08F 220/14, C08J 5/00, C08K 5/01, C08K 5/101, C08L 33/12

(54) **MONOMER COMPOSITION, RESIN COMPOSITION, METHOD FOR PRODUCING RESIN COMPOSITION, RESIN MOLDED BODY, AND METHOD FOR PRODUCING RESIN MOLDED BODY**

(30) Priority: 18.11.2022 JP 2022184555
(71) Applicant: Mitsubishi Chemical Corporation, Tokyo 100-8251 (JP)
(72) Inventor: FURUYA, Kenji, Tokyo 100-8251 (JP); HIRANO, Yusuke, Tokyo 100-8251 (JP); ISOMURA, Manabu, Tokyo 100-8251 (JP); KANEMORI, Kouichi, Tokyo 100-8251 (JP)
(74) Representative: Ter Meer Steinmeister & Partner
(86) International application number: PCT/JP2023/041381
(87) International publication number: WO 2024/106522

(57) **Abstract**

A monomer composition includes methyl methacrylate, an α-olefin, and at least one compound selected from the group consisting of methyl propionate, methyl pyruvate, and methyl 2-methylbutyrate. In the monomer composition, the total content of the methyl propionate, the methyl pyruvate, and the methyl 2-methylbutyrate is more than 36 ppm by mass with respect to the total mass of the monomer composition, and the α-olefin includes at least one selected from the group consisting of 2-ethyl-1-hexene, 1-octene, and 1-dodecene.

## Description

### TECHNICAL FIELD

The present invention relates to a monomer composition, a resin composition, a method of producing a resin composition, a resin molded body, and a method of producing a resin molded body.

### BACKGROUND ART

Methacrylic resins are excellent in transparency, heat resistance, and weather resistance, and have an ability having a balance of the physical properties of the resins, such as mechanical strength, thermal properties, and molding workability. In view of such excellent characteristics, the resins have been used in many applications such as vehicular members, medical members, toys, liquid containers, optical materials, signboards, displays, decorative members, building members, and the face plates of electronic instruments, and particularly used in members having light-transmitting properties.

In the applications described above, there has been a problem that the transparency of the methacrylic resin plates is greatly deteriorated in a case in which members employing methacrylic resin plates are placed under environments exposed to direct sunlight, and light from UV lamps and the like. Therefore, a methacrylic resin of which the transparency is maintained even if the methacrylic resin is exposed to light for a long time, that is, a methacrylic resin excellent in light stability has been demanded.

For example, Patent Document 1 discloses a methacrylic resin obtained by polymerizing a monomer such as methyl methacrylate in the presence of a hindered amine compound having a specific structure, which is one of light stabilizers, for a technology to improve the light stability of the methacrylic resin. Patent Document 2 discloses a methacrylic resin including a polymer including a triazine-based compound as a structural unit.

### PRIOR ART DOCUMENTS

### PATENT DOCUMENTS

Patent Document 1: Japanese Unexamined Patent Application Publication No. S55-139404
Patent Document 2: Japanese Unexamined Patent Application Publication No. 2012-72333

### SUMMARY OF INVENTION

### TECHNICAL PROBLEM

However, the methacrylic resins described in Patent Documents 1 and 2 have had a problem that with increasing the amount of an additive in polymerization, coloration resulting from the additive occurs while light stability is improved. Therefore, there is a problem in that it has been impossible to use the methacrylic resins in the case of simultaneously demanding a specific tone and transparency.

Under the circumstances described above, an objective of the present invention is to provide: a monomer composition for obtaining a resin composition excellent in light stability while maintaining transparency and heat resistance inherent to a methacrylic resin; a resin composition; a method of producing a resin composition; a resin molded body; and a method of producing a resin molded body.

### SOLUTION TO PROBLEM

The present invention has the following features in order to solve the problems described above.
[1] A monomer composition comprising methyl methacrylate, an α-olefin, and at least one compound selected from the group consisting of methyl propionate, methyl pyruvate, and methyl 2-methylbutyrate, wherein
   a total content of the methyl propionate, the methyl pyruvate, and the methyl 2-methylbutyrate is more than 36 ppm by mass with respect to a total mass of the monomer composition, and
   the α-olefin comprises at least one selected from the group consisting of 2-ethyl-1-hexene, 1-octene, and 1-dodecene.
[2] The monomer composition according to [1], wherein a content of the methyl methacrylate is 85% by mass or more with respect to the total mass of the monomer composition.
[3] The monomer composition according to [1] or [2], wherein a content of the methyl methacrylate is 90% by mass or more with respect to the total mass of the monomer composition.
[4] The monomer composition according to any one of [1] to [3], wherein a content of the α-olefin is 0.1 ppm by mass or more with respect to the total mass of the monomer composition.
[5] The monomer composition according to any one of [1] to [4], wherein a content of the α-olefin is 10 ppm by mass or more with respect to the total mass of the monomer composition.
[6] The monomer composition according to any one of [1] to [5], wherein a content of the α-olefin is 60 ppm by mass or more with respect to the total mass of the monomer composition.
[7] The monomer composition according to any one of [1] to [6], wherein a content of the α-olefin is 80 ppm by mass or more with respect to the total mass of the monomer composition.
[8] The monomer composition according to any one of [1] to [7], wherein a content of at least one compound selected from the group consisting of a transition metal compound and a Group 13 element compound is 2 × 10⁴ ppm by mass or less with respect to the total mass of the α-olefin.
[9] The monomer composition according to any one of [1] to [8], wherein the total content of the methyl propionate, the methyl pyruvate, and the methyl 2-methylbutyrate is 40 ppm by mass or more with respect to the total mass of the monomer composition.
[10] The monomer composition according to any one of [1] to [9], wherein the total content of the methyl propionate, the methyl pyruvate, and the methyl 2-methylbutyrate is 60 ppm by mass or more with respect to the total mass of the monomer composition.
[11] The monomer composition according to any one of [1] to [10], wherein the total content of the methyl propionate, the methyl pyruvate, and the methyl 2-methylbutyrate is 70 ppm by mass or more with respect to the total mass of the monomer composition.
[12] The monomer composition according to any one of [1] to [11], wherein the total content of the methyl propionate, the methyl pyruvate, and the methyl 2-methylbutyrate is 100 ppm by mass or more with respect to the total mass of the monomer composition.
[13] The monomer composition according to any one of [1] to [12], wherein a ratio of a content of the α-olefin to the total content of the methyl propionate, the methyl pyruvate, and the methyl 2-methylbutyrate ([a mass of the α-olefin]/[a total mass of the methyl propionate, the methyl pyruvate, and the methyl 2-methylbutyrate]) is 0.00001 or more and 1000 or less.
[14] The monomer composition according to any one of [1] to [13], further comprising an acrylic ester.
[15] The monomer composition according to [14], wherein the acrylic ester is at least one compound selected from the group consisting of methyl acrylate, ethyl acrylate, and n-butyl acrylate.
[16] The monomer composition according to [14], wherein the acrylic ester is methyl acrylate or ethyl acrylate.
[17] The monomer composition according to any one of [1] to [16], further comprising styrene.
[18] The monomer composition according to any one of [1] to [17], further comprising methyl isobutyrate.
[19] A method of producing a resin composition, the method comprising a radical polymerization step of performing radical polymerization of a polymerizable composition comprising the monomer composition according to any one of [1] to [18].
[20] A resin composition comprising a polymer of the monomer composition according to any one of [1] to [18].
[21] A resin composition comprising a methacrylic polymer (P), an α-olefin, and at least one compound selected from the group consisting of methyl propionate, methyl pyruvate, and methyl 2-methylbutyrate, wherein
   a total content of the methyl propionate, the methyl pyruvate, and the methyl 2-methylbutyrate is more than 26 ppm by mass with respect to a total mass of the resin composition, and
   the α-olefin comprises at least one selected from the group consisting of 2-ethyl-1-hexene, 1-octene, and 1-dodecene.
[22] The resin composition according to [21], wherein the methacrylic polymer (P) comprises 50% by mass or more of a repeating unit derived from methyl methacrylate with respect to a total mass of the methacrylic polymer (P).
[23] The resin composition according to [21] or [22], wherein the methacrylic polymer (P) comprises 70% by mass or more of a repeating unit derived from methyl methacrylate with respect to a total mass of the methacrylic polymer (P).
[24] The resin composition according to any one of [21] to [23], wherein a content of the α-olefin is 0.1 ppm by mass or more with respect to the total mass of the resin composition.
[25] The resin composition according to any one of [21] to [24], wherein a content of the α-olefin is 10 ppm by mass or more with respect to the total mass of the resin composition.
[26] The resin composition according to any one of [21] to [25], wherein a content of the α-olefin is 60 ppm by mass or more with respect to the total mass of the resin composition.
[27] The resin composition according to any one of [21] to [26], wherein a content of the α-olefin is 80 ppm by mass or more with respect to the total mass of the resin composition.
[28] The resin composition according to any one of [21] to [27], wherein the total content of the methyl propionate, the methyl pyruvate, and the methyl 2-methylbutyrate is 30 ppm by mass or more with respect to the total mass of the resin composition.
[29] The resin composition according to any one of [21] to [28], wherein the total content of the methyl propionate, the methyl pyruvate, and the methyl 2-methylbutyrate is 40 ppm by mass or more with respect to the total mass of the resin composition.
[30] The resin composition according to any one of [21] to [29], wherein the total content of the methyl propionate, the methyl pyruvate, and the methyl 2-methylbutyrate is 60 ppm by mass or more with respect to the total mass of the resin composition.
[31] The resin composition according to any one of [21] to [30], wherein the total content of the methyl propionate, the methyl pyruvate, and the methyl 2-methylbutyrate is 80 ppm by mass or more with respect to the total mass of the resin composition.
[32] The resin composition according to any one of [21] to [31], further comprising methyl isobutyrate in the resin composition.
[33] The resin composition according to any one of [21] to [32], wherein the methacrylic polymer (P) comprises a repeating unit derived from methyl methacrylate and a repeating unit derived from an acrylic ester.
[34] The resin composition according to any one of [21] to [32], wherein the methacrylic polymer (P) comprises a repeating unit derived from methyl methacrylate and a repeating unit derived from styrene.
[35] A resin molded body comprising the resin composition according to any one of [20] to [34].
[36] A vehicular member comprising the resin molded body according to [35].
[37] A medical member comprising the resin molded body according to [35].
[38] A toy comprising the resin molded body according to [35].
[39] A liquid container comprising the resin molded body according to [35].
[40] An optical material comprising the resin molded body according to [35].
[41] A signboard comprising the resin molded body according to [35].
[42] A display comprising the resin molded body according to [35].
[43] A method of producing a resin molded body, wherein
   the method comprises a molding step of molding a resin composition comprising a methacrylic polymer (P), an α-olefin, and at least one compound selected from the group consisting of methyl propionate, methyl pyruvate, and methyl 2-methylbutyrate,
   a total content of the methyl propionate, the methyl pyruvate, and the methyl 2-methylbutyrate in the resin composition is more than 26 ppm by mass with respect to a total mass of the resin composition, and
   the α-olefin comprises at least one selected from the group consisting of 2-ethyl-1-hexene, 1-octene, and 1-dodecene.

### ADVANTAGEOUS EFFECTS OF INVENTION

In accordance with the present invention, there can be provided: a monomer composition for obtaining a resin composition excellent in light stability while maintaining transparency and heat resistance inherent to a methacrylic resin; a resin composition; a method of producing a resin composition; a resin molded body; and a method of producing a resin molded body.

### DESCRIPTION OF EMBODIMENTS

The definitions of the following terms in the present specification and claims are as follows.

The term "monomer" means a compound having a polymerizable carbon-carbon double bond.

The term "repeating unit" means a unit that is formed by polymerizing a monomer and derived from the monomer. The repeating unit may be a unit that is directly formed by polymerization reaction, or may be a unit obtained by converting a part of the unit into another structure by treating a polymer.

The term "(meth)acrylate" means either or both of "acrylate" and "methacrylate".

The term "(meth)acryloyl" means either or both of "acryloyl" and "methacryloyl".

The term "(meth)acrylic acid" means either or both of "acrylic acid" and "methacrylic acid".

The term "conjugation" means overlapping of p-orbitals across an intervening sigma bond.

The term "nonconjugation" means that conjugation does not occur.

The term "obtained resin composition" means a resin composition obtained by radical polymerization of a mixture of monomers, including a monomer composition.

The term "obtained resin molded body" means a resin molded body obtained by molding a resin composition.

The term "% by mass" denotes the content rate of a predetermined component included in a total amount of 100% by mass.

The term "mass average molecular weight" is a value measured by using gel permeation chromatography using standard polystyrene as a standard sample.

The terms "UV" and "ultraviolet" mean light principally including light in a wavelength range of 295 to 430 nm.

The term "transition metal" means any of metal elements within Groups 3 to 12 in the periodic table. Typical examples include scandium (Sc), titanium (Ti), vanadium (V), chromium (Cr), manganese (Mn), iron (Fe), cobalt (Co), nickel (Ni), copper (Cu), zinc (Zn), yttrium (Y), zirconium (Zr), niobium (Nb), molybdenum (Mo), technetium (Tc), ruthenium (Ru), rhodium (Rh), palladium (Pd), silver (Ag), cadmium (Cd), lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), hafnium (Hf), tantalum (Ta), tungsten (W), rhenium (Re), osmium (Os), iridium (Ir), platinum (Pt), gold (Au), and mercury (Hg).

The term "Group 13 element" means any of elements within Group 13 in the periodic table. Typical examples thereof include boron (B), aluminum (Al), gallium (Ga), indium (In), and thallium (T1).

The term "periodic table" means "Periodic Table of Elements" ("Periodic Table of Elements", [online], National Center for Biotechnology Information, [searched on November 7, 2022], the Internet, <URL: https://pubchem.ncbi.nlm.nih.gov/periodic-table/>).

In the present specification, a numerical range expressed by "x to y" means a range including the values of x and y as the lower and upper limit values, respectively, and the term "A to B" means that A or more and B or less.

### <1. Monomer Composition>

A monomer composition according to a first embodiment of the present invention includes methyl methacrylate, an α-olefin, and at least one compound selected from the group consisting of propionate, methyl pyruvate, and methyl 2-methylbutyrate, wherein the total content of the methyl propionate, the methyl pyruvate, and the methyl 2-methylbutyrate is more than 36 ppm by mass with respect to the total mass of the monomer composition, and the α-olefin includes at least one selected from the group consisting of 2-ethyl-1-hexene, 1-octene, and 1-dodecene (hereinafter, "α-olefin" refers to at least one selected from the group consisting of 2-ethyl-1-hexene, 1-octene, and 1-dodecene, unless otherwise specified). Another component may also be included as long as the effects of the present invention are not impaired.

### <1-1. Methyl Methacrylate>

The monomer composition according to the present embodiment includes methyl methacrylate. The methyl methacrylate can be produced by a method such as, for example, an acetone cyanohydrin method, a new acetone cyanohydrin method, a C4 direct oxidation method, a direct methyl esterification method, an ethylene method, or a new ethylene method. Methyl methacrylate obtained by thermally decomposing a resin composition obtained by polymerizing a monomer composition including methyl methacrylate may also be used. The methyl methacrylate is more preferably methyl methacrylate obtained by thermally decomposing a resin composition obtained by polymerizing a monomer composition including methyl methacrylate. The monomer composition according to the present embodiment includes methyl methacrylate, whereby a resin composition having excellent light stability and heat resistance that is inherent to a methacrylic resin can be provided.

The lower limit of the content of the methyl methacrylate with respect to the total mass of the monomer composition is not particularly limited, and is preferably 85% by mass or more, more preferably 90% by mass or more, still more preferably 95% by mass or more, and particularly preferably 97% by mass or more. The upper limit of the content of the methyl methacrylate is not particularly limited, is typically 99.99% by mass or less, and may be 99.98% by mass or less, or 99.97% by mass or less. Accordingly, examples thereof include ranges of 85% by mass or more and 99.99% by mass or less, 90% by mass or more and 99.98% by mass or less, 95% by mass or more and 99.97% by mass or less, 97% by mass or more and 99.97% by mass or less, and 97% by mass or more and 99.97% by mass or less.

The total content of the methyl methacrylate, the α-olefin, and at least one compound selected from the group consisting of the methyl propionate, the methyl pyruvate, and the methyl 2-methylbutyrate with respect to the total mass of the monomer composition according to the present embodiment is not particularly limited, and is typically 100% by mass or less.

### <1-2. α-Olefin>

The monomer composition according to the present embodiment includes an α-olefin including at least one selected from the group consisting of 2-ethyl-1-hexene, 1-octene, and 1-dodecene, whereby a resin composition having excellent light stability can be provided. Such α-olefins may be used singly, or in optional combination of two or more kinds thereof at an optional ratio.

The lower limit of the content of the α-olefin with respect to the total mass of the monomer composition according to the present embodiment is not particularly limited, and is preferably 0.1 ppm by mass or more, more preferably 10 ppm by mass or more, still more preferably 60 ppm by mass or more, even more preferably 80 ppm by mass or more, and particularly preferably 100 ppm by mass or more because a resin composition having favorable light stability can be provided.

The upper limit of the content of the α-olefin in the monomer composition according to the present embodiment is not particularly limited, and is preferably 10000 ppm by mass or less, more preferably 5000 ppm by mass or less, still more preferably 4000 ppm by mass or less, even more preferably 3000 ppm by mass or less, and particularly preferably 2000 ppm by mass or less because a resin composition enabling heat resistance to be favorably maintained can be provided.

The preferred upper and lower limit values described above can be optionally combined.

Specifically, the content of the α-olefin with respect to the total mass of the monomer composition according to the present embodiment is preferably 0.1 ppm by mass or more and 10000 ppm by mass or less, more preferably 10 ppm by mass or more and 5000 ppm by mass or less, still more preferably 60 ppm by mass or more and 4000 ppm by mass or less, even more preferably 80 ppm by mass or more and 3000 ppm by mass or less, and particularly preferably 100 ppm by mass or more and 2000 ppm by mass or less. In a case in which the monomer composition includes two or more kinds of α-olefins, the content described above is the total content of the two or more kinds of α-olefins.

The α-olefin is presumed to be excellent in the effect of scavenging radicals in view of a stable coupling product of the olefins to which radicals generated by ultraviolet rays are added.

2-Ethyl-1-hexene, 1-octene, and 1-dodecene easily remain in the obtained resin composition without volatilizing due to heating in the polymerization. Therefore, 2-ethyl-1-hexene, 1-octene, and 1-dodecene can sufficiently contribute to improvement in the light stability of the resin composition obtained by the polymerization of the monomer composition. A small content of at least one α-olefin selected from the group consisting of 2-ethyl-1-hexene, 1-octene, and 1-dodecene easily exerts the effect of improving light stability. As described later, hydrogen bound to carbon adjacent to a double bond is considered to be involved in the effect of improving light stability by the α-olefin. Therefore, a decrease in the number of carbon atoms under the same mass condition results in an increase in the number of carbon atoms involved in a double bond in one α-olefin molecule, and is therefore considered to facilitate the exertion of the effect of improving light stability.

Among 2-ethyl-1-hexene, 1-octene and 1-dodecene, the α-olefin is more preferably one or more selected from 1-octene and 2-ethyl-1-hexene, and still more preferably 1-octene because 1-octene easily remains in the resin composition after the polymerization. The rate of 1-octene in the α-olefin is not particularly limited, and is preferably 50% by mass or more, more preferably 80% by mass or more, still more preferably 90% by mass or more, and typically 100% by mass or less with respect to the total mass of the α-olefin.

In the monomer composition according to the present embodiment, the content of at least one compound selected from the group consisting of a transition metal compound and a Group 13 element compound is preferably 0 ppm by mass or more and 2 × 10⁴ ppm by mass or less with respect to the total mass of the α-olefin.

The α-olefin in the present embodiment precludes the obtainment of a resonance stabilization effect and has considerably low reactivity in comparison with methyl methacrylate which is a conjugate monomer. Therefore, an unreacted α-olefin (hereinafter also referred to as "α-olefin monomer") remains in the obtained resin composition unless under special conditions of using a specific polymerization catalyst, such as at least one compound selected from the group consisting of the transition metal compound and the Group 13 element compound, to exert the effect of the catalyst. The remaining of the α-olefin monomer in the resin composition is considered to enable the provision of a resin composition having favorable light stability. In other words, for preventing the at least one compound from exerting the effect of the catalyst, the content of the at least one compound is preferably 2 × 10⁴ ppm by mass or less, more preferably 1 × 10⁴ ppm by mass or less, still more preferably 1000 ppm by mass or less, and particularly preferably 500 ppm by mass or less with respect to the total mass of the α-olefin, and it is particularly preferable that the at least one compound is not included. Here, the term "not included" means that the content is less than a detection limit.

Examples of the kind of the at least one compound include: compounds of the transition metals of Groups 5 to 11 having chelating ligands; and Lewis acid catalysts. Specific examples of the transition metals include vanadium, niobium, tantalum, chromium, molybdenum, tungsten, manganese, iron, platinum, ruthenium, cobalt, rhodium, nickel, palladium, and copper. Of these, the transition metals are preferably the transition metals of Groups 8 to 11, more preferably the transition metals of Group 10, still more preferably nickel (Ni) or palladium (Pd). These transition metals may be used singly, or in combination of two or more kinds thereof.

Each of the chelating ligands has at least two atoms selected from the group consisting of P, N, O, and S, includes a bidentate or multidentate ligand, and is electronically neutral or anionic. In a review by Ittel et al., an example of the structure of a chelating ligand is described (Ittel et al., "Late-Metal Catalysts for Ethylene Homo- and Copolymerization", Chemical Reviews, March 25, 2000, vol. 100, No. 4, pp. 1169-1204). Examples of the chelating ligand include bidentate anionic P and O ligands. Examples of the bidentate anionic P and O ligands include phosphorus sulfonate, phosphorus carboxylate, phosphorus phenol, and phosphorus enolate. Examples of chelating ligands other than the bidentate anionic P and O ligands include bidentate anionic N and O ligands. Examples of the bidentate anionic N and O ligands include salicylaldiminato and pyridinecarboxylic acid. Examples of chelating ligands other than the bidentate anionic P and O ligands and the bidentate anionic N and O ligands include diimine ligands, diphenoxide ligands, and diamide ligands.

Typically, catalysts such as, so-called, SHOP-based catalysts and Drent-based catalysts are known as catalysts which are compounds of transition metals of Groups 5 to 11 having the chelating ligands. Such a SHOP-based catalyst as described above is a catalyst in which a phosphorus-based ligand having an aryl group which may have a substituent is coordinated to nickel metal. Such a Drent-based catalyst as described above is a catalyst in which a phosphorus-based ligand having an aryl group which may have a substituent is coordinated to palladium metal.

Typical examples of the Lewis acid catalysts include cationic complexes of divalent palladium or platinum. The cationic complexes of divalent palladium or platinum exhibit Lewis acidity, and are useful as Lewis acid catalysts for Diels-Alder reactions and the like. For example, the Group 13 element compound such as boron and aluminum, the 4th period transition metal such as titanium, and the 5th period transition metal such as zirconium also exhibit Lewis acidity, and are therefore preferred.

### <1-3. At Least One Compound Selected from Group Consisting of Methyl Propionate, Methyl Pyruvate, and Methyl 2-Methylbutyrate>

At least one compound selected from the group consisting of methyl propionate, methyl pyruvate, and methyl 2-methylbutyrate is one of the components included in the monomer composition according to the present embodiment. The total content of the methyl propionate, the methyl pyruvate, and the methyl 2-methylbutyrate with respect to the total mass of the monomer composition is allowed to be more than 36 ppm by mass, whereby a resin composition having excellent in light stability can be provided. In a case in which the monomer composition includes two or more compounds selected from the group consisting of methyl propionate, methyl pyruvate, and methyl 2-methylbutyrate, the above-described content means the total content of these compounds. For specific examples, the content of one compound may be more than 36 ppm by mass in the case of the inclusion of only one compound of the three compounds, and the total content of the three compounds may be more than 36 ppm by mass in the case of the inclusion of all the three compounds.

The lower limit of the total content of the methyl propionate, the methyl pyruvate, and the methyl 2-methylbutyrate with respect to the total mass of the monomer composition according to the present embodiment is not particularly limited, and is typically more than 36 ppm by mass, preferably 40 ppm by mass or more, more preferably 60 ppm by mass or more, still more preferably 70 ppm by mass or more, even more preferably 80 ppm by mass or more, and particularly preferably 100 ppm by mass or more because a resin composition having favorable light stability can be provided.

The upper limit of the total content of the methyl propionate, the methyl pyruvate, and the methyl 2-methylbutyrate with respect to the total mass of the monomer composition according to the present embodiment is not particularly limited, and is preferably 20000 ppm by mass or less, more preferably 15000 ppm by mass or less, still more preferably 10000 ppm by mass or less, even more preferably 6000 ppm by mass or less, and particularly preferably 5000 ppm by mass or less because a resin composition enabling heat resistance to be favorably maintained can be provided.

The preferred upper and lower limit values described above can be optionally combined.

Specifically, the total content of the methyl propionate, the methyl pyruvate, and the methyl 2-methylbutyrate in the monomer composition according to the present embodiment is preferably more than 36 ppm by mass and 20000 ppm by mass or less, more preferably 40 ppm by mass or more and 20000 ppm by mass or less, still more preferably 60 ppm by mass or more and 15000 ppm by mass or less, even more preferably 70 ppm by mass or more and 10000 ppm by mass or less, particularly preferably 80 ppm by mass or more and 6000 ppm by mass or less, and most preferably 100 ppm by mass or more and 5000 ppm by mass or less.

In the monomer composition according to the present embodiment, the upper limit of the ratio of the content of the α-olefin to the total content of the methyl propionate, the methyl pyruvate, and the methyl 2-methylbutyrate (also referred to as "ratio of [mass of α-olefin]/[total mass of methyl propionate, methyl pyruvate, and methyl 2-methylbutyrate]") is not particularly limited, and is preferably 1000 or less, more preferably 500 or less, still more preferably 300 or less, even more preferably 100 or less, and particularly preferably 10 or less in view of enabling a resin molded body having favorable light stability to be provided by the interaction between at least one compound selected from the group consisting of the methyl propionate, the methyl pyruvate, and the methyl 2-methylbutyrate, and the α-olefin. The lower limit of the ratio of [mass of α-olefin]/[total mass of methyl propionate, methyl pyruvate, and methyl 2-methylbutyrate] is not particularly limited, and is preferably 0.00001 or more, more preferably 0.0001 or more, still more preferably 0.001 or more, even more preferably 0.01 or more, and particularly preferably 0.5 or more from the viewpoint of allowing the heat resistance of the resin molded body to be favorable.

The upper and lower limit values described above can be optionally combined. Examples of the preferred range of the ratio of [mass of α-olefin]/[total mass of methyl propionate, methyl pyruvate, and methyl 2-methylbutyrate] include ranges of 0.00001 or more and 1000 or less, 0.0001 or more and 500 or less, 0.001 or more and 300 or less, 0.01 or more and 100 or less, and 0.5 or more and 10 or less. Of these, the ratio of [mass of α-olefin]/[total mass of methyl propionate, methyl pyruvate, and methyl 2-methylbutyrate] is more preferably 0.0001 or more and 500 or less, and still more preferably 0.001 or more and 300 or less.

### <1-4. Monomers Other Than Methyl Methacrylate>

The monomer composition according to the present embodiment may include a monomer other than the methyl methacrylate. In the present specification, "monomer" means an unpolymerized compound. Examples of the monomer other than the methyl methacrylate include monomers described in the following (1) to (16). The monomers described in the following (1) to (16) may be used singly, or in optional combination of two or more kinds thereof at an optional ratio.
(1) Methacrylic ester:
   For example, ethyl methacrylate, isopropyl methacrylate, n-butyl methacrylate, iso-butyl methacrylate, tert-butyl methacrylate, 2-ethylhexyl methacrylate, phenyl methacrylate, or benzyl methacrylate.
(2) Acrylic ester:
   For example, methyl acrylate, ethyl acrylate, n-butyl acrylate, iso-butyl acrylate, tert-butyl acrylate, or 2-ethylhexyl acrylate.
(3) Unsaturated carboxylic acid:
   For example, acrylic acid, methacrylic acid, maleic acid, or itaconic acid.
(4) Unsaturated carboxylic acid anhydride:
   For example, maleic anhydride or itaconic anhydride.
(5) Maleimide:
   For example, N-phenylmaleimide or N-cyclohexylmaleimide.
(6) Vinyl monomer containing hydroxy group:
   For example, 2-hydroxyethyl acrylate, 2-hydroxyethyl methacrylate, or 2-hydroxypropyl methacrylate.
(7) Vinyl ester:
   For example, vinyl acetate or vinyl benzoate.
(8) Vinyl chloride, vinylidene chloride, or derivatives thereof.
(9) Vinyl monomer containing nitrogen:
   For example, methacrylamide or acrylonitrile.
(10) Monomer containing epoxy group:
   For example, glycidyl acrylate or glycidyl methacrylate.
(11) Aromatic vinyl monomer:
   For example, styrene or α-methylstyrene.
(12) Alkanediol di(meth)acrylate:
   For example, ethylene glycol di(meth)acrylate, 1,2-propylene glycol di(meth)acrylate, 1,3-butylene glycol di(meth)acrylate, neopentyl glycol di(meth)acrylate, or 1,6-hexanediol di(meth)acrylate.
(13) Polyalkylene glycol di(meth)acrylate:
   For example, diethylene glycol di(meth)acrylate, dipropylene glycol di(meth)acrylate, triethylene glycol (meth)acrylate, tetraethylene glycol di(meth)acrylate, or polyethylene glycol di(meth)acrylate.
(14) Vinyl monomer having two or more ethylenically unsaturated bonds in the molecule:
   For example, divinylbenzene.
(15) Unsaturated polyester prepolymer obtained from at least one polyvalent carboxylic acid including ethylenically unsaturated polycarboxylic acid and at least one diol.
(16) Vinyl ester prepolymer obtained by acrylic modification of terminal of epoxy group.

Of these, the monomer is preferably at least one acrylic ester selected from the group consisting of methyl acrylate, ethyl acrylate, and n-butyl acrylate, and more preferably methyl acrylate or ethyl acrylate, in view of enabling the provision of a resin composition having an excellent balance of transparency, heat resistance, and moldability. The content of the acrylic ester is preferably 0% by mass or more and 30% by mass or less with respect to the total mass of the monomer composition. The inclusion of an acrylic ester in the monomer composition enables a resin composition having excellent light stability to be provided. Moreover, it is possible to suppress the deterioration of light stability in a case in which a resin molded body including the resin composition is exposed to a light for a long time. Moreover, the change of the acrylic ester to styrene also enables application to the production of a methacrylic polymer (P1) containing a repeating unit derived from methyl methacrylate (hereinafter also referred to as "methyl methacrylate unit") and a repeating unit derived from styrene (hereinafter also referred to as "styrene unit"). In such a case, the content rate of a styrene unit described in <3-2. Methacrylic Polymer (P)> can be applied as the content of styrene.

### <1-5. Methyl Isobutyrate>

The monomer composition preferably further includes methyl isobutyrate. The inclusion of the compound enables a resin composition having further excellent light stability to be provided. This is presumed to further improve the effect of improving light stability due to the inclusion of an α-olefin and a certain amount of at least one compound selected from the group consisting of methyl propionate, methyl pyruvate, and methyl 2-methylbutyrate, described in <3-1. Operation-Effects>. Moreover, the deterioration of light stability in a case in which a resin molded body including the resin composition is exposed to light for a long time can be suppressed.

In a case in which the monomer composition according to the present embodiment includes methyl isobutyrate, the total content of the methyl isobutyrate, methyl propionate, methyl pyruvate, and methyl 2-methylbutyrate with respect to the total mass of the monomer composition is preferably an amount in the range of the total content of the methyl propionate, the methyl pyruvate, and the methyl 2-methylbutyrate, described above.

In a case in which the monomer composition according to the present embodiment includes methyl isobutyrate, the content of the methyl isobutyrate with respect to the total mass of the monomer composition is preferably 10 ppm by mass or more, more preferably 50 ppm by mass or more, still more preferably 100 ppm by mass or more, particularly preferably 200 ppm by mass or more, and most preferably 250 ppm by mass or more, and preferably 10000 ppm by mass or less, more preferably 8000 ppm by mass or less, still more preferably 5000 ppm by mass or less, particularly preferably 3000 ppm by mass or more, and most preferably 1500 ppm by mass or more.

The preferred upper and lower limit values described above can be optionally combined. Specific examples of the preferred range of the content of the methyl isobutyrate with respect to the total mass of the monomer composition according to the present embodiment include ranges of 10 ppm by mass or more and 10000 ppm by mass or less, 50 ppm by mass or more and 8000 ppm by mass or less, 100 ppm by mass or more and 5000 ppm by mass or less, 200 ppm by mass or more and 3000 ppm by mass or less, and 250 ppm by mass or more and 1500 ppm by mass or less.

### <1-6. Additive>

The monomer composition according to the present embodiment may include another additive. Examples of the additive include known additives such as mold release agents, thermal stabilizers, lubricants, plasticizers, antioxidants, antistatic agents, light stabilizers (other than α-olefins, methyl isobutyrate, methyl propionate, methyl pyruvate, and methyl 2-methylbutyrate), ultraviolet absorbents, flame retardants, fire retarding aids, fillers, pigments, dyes, silane coupling agents, leveling agents, antifoaming agents, and fluorescent agents. Such additives as described above may be used singly, or in optional combination of two or more kinds thereof at an optional ratio.

In the present embodiment, the α-olefin and at least one compound selected from the group consisting of the methyl propionate, the methyl pyruvate, and the methyl 2-methylbutyrate are considered to exhibit excellent light stability due to the mechanism of action different from those of commonly known UV absorbents and radical scavengers (HALSs). Thus, the α-olefin and at least one compound selected from the group consisting of the methyl propionate, the methyl pyruvate, and the methyl 2-methylbutyrate can also be used together with an additive such as a UV absorbent or HALS. The monomer composition includes the α-olefin, at least one compound selected from the group consisting of the methyl propionate, the methyl pyruvate, and the methyl 2-methylbutyrate, and the additive, whereby a resin composition and a resin molded body having greater light stability can be provided.

The monomer composition according to the present embodiment may also include a compound, inevitably incorporated into methyl methacrylate, such as methacrolein or methanol.

### <2. Polymerizable Composition>

A polymerizable composition according to a second embodiment of the present invention is one aspect of a raw material for obtaining a resin composition according to a third embodiment of the present invention, described later. The polymerizable composition according to the present embodiment (also referred to as "polymerizable composition (X2)") is, for example, a polymerizable composition including the monomer composition and, if necessary, a known radical polymerization initiator.

### <2-1. Radical Polymerization Initiator>

Examples of the radical polymerization initiator include: known azo compounds such as 2,2'-azobis(isobutyronitrile) and 2,2'-azobis(2,4-dimethylvaleronitrile); and known organic peroxides such as benzoyl peroxide and lauroyl peroxide. These may be used singly, or in optional combination of two or more kinds thereof at an optional ratio. If necessary, a known polymerization promoter such as an amine or a mercaptan can be used together with the radical polymerization initiator.

The content of the radical polymerization initiator in the polymerizable composition (X2) is not particularly limited, and can be determined as appropriate by those skilled in the art according to well-known techniques. Specifically, the content of the radical polymerization agent may be 0.005 part by mass or more and 5 parts by mass or less, or 0.01 part by mass or more and 1.0 part by mass or less with respect to 100 parts by mass of the total mass of the polymerizable composition (X2).

### <2-2. Additive>

The mode of an additive is similar to that described in <1-6. Additive>.

The additive may be one type or two or more types.

### <3. Resin Composition>

The resin composition according to the third embodiment of the present invention (hereinafter also simply referred to as "resin composition") is a resin composition including at least a methacrylic polymer (P), an α-olefin, and at least one compound selected from the group consisting of methyl propionate, methyl pyruvate, and methyl 2-methylbutyrate, wherein the total content of the methyl propionate, the methyl pyruvate, and the methyl 2-methylbutyrate is more than 26 ppm by mass with respect to the total mass of the resin composition, and the α-olefin includes at least one selected from the group consisting of 2-ethyl-1-hexene, 1-octene, and 1-dodecene. In a case in which the resin composition includes two or more compounds selected from the group consisting of the methyl propionate, the methyl pyruvate, and the methyl 2-methylbutyrate, the above-described content means the total content of these compounds. For specific examples, the content of one compound may be more than 26 ppm by mass in the case of the inclusion of only one compound of the three compounds, and the total content of the three compounds may be more than 26 ppm by mass in the case of the inclusion of all the three compounds. The resin composition according to the present embodiment may be a composition including a polymer of the monomer composition according to the first embodiment of the present invention, or may be a composition obtained by radical polymerization of the polymerizable composition according to the second embodiment of the present invention.

The resin composition according to the present embodiment includes the methacrylic polymer (P), whereby a resin molded body having excellent heat resistance and favorable transparency can be provided.

The resin composition includes an α-olefin and a specific content of at least one compound selected from the group consisting of methyl propionate, methyl pyruvate, and methyl 2-methylbutyrate in the resin composition including the methacrylic polymer (P), so that the α-olefin and at least one compound selected from the group consisting of the methyl propionate, the methyl pyruvate, and the methyl 2-methylbutyrate are present in the polymerization chain of the methacrylic polymer (P) in a monomeric state, and it is possible to provide a resin molded body in which a yellow tinge is inhibited from being generated even when exposed to UV for a long time, and the deterioration of light stability is suppressed. The form of the resin composition is not particularly limited, and is commonly a solid.

The mode of at least one compound selected from the group consisting of the methyl propionate, the methyl pyruvate, and the methyl 2-methylbutyrate is similar to that described in <1-3. At Least One Compound Selected from Group Consisting of Methyl Propionate, Methyl Pyruvate, And Methyl 2-Methylbutyrate>. The mode of the α-olefin is similar to that described in <1-2. α-Olefin>.

The content of the methacrylic polymer (P) with respect to the total mass of the resin composition is not particularly limited, and is commonly 80.0% by mass or more, preferably 85.0% by mass or more, more preferably 90.0% by mass or more, still more preferably 95.0% by mass or more, and particularly preferably 99.0% by mass or more from the viewpoint of allowing heat resistance to be favorable. On the other hand, the content is typically 99.99% by mass or less, preferably 99.9785% by mass or less, more preferably 99.97% by mass or less, still more preferably 99.95% by mass or less, and particularly preferably 99.90% by mass or less from the viewpoint of obtaining excellent light stability. The upper and lower limit values described above can be optionally combined. Preferred examples of the range of the content of the methacrylic polymer (P) include ranges of 80.0% by mass or more and 99.99% by mass or less, 85.0% by mass or more and 99.9785% by mass or less, 90.0% by mass or more and 99.97% by mass or less, 95.0% by mass or more and 99.95% by mass or less, and 99.0% by mass or more and 99.90% by mass or less. In a case in which the resin composition includes two or more kinds of methacrylic polymers (P), the content described above is the total content of the two or more kinds of methacrylic polymers (P).

The content of the α-olefin with respect to the total mass of the resin composition is not particularly limited. The content of the α-olefin is typically 0.1 ppm by mass or more, preferably 10 ppm by mass or more, more preferably 60 ppm by mass or more, still more preferably 80 ppm by mass or more, and particularly preferably 100 ppm by mass or more from the viewpoint of obtaining excellent light stability.

The upper limit of the content of the α-olefin with respect to the total mass of the resin composition according to the present embodiment is not particularly limited, and is typically 10000 ppm by mass or less, preferably 5000 ppm by mass or less, more preferably 4000 ppm by mass or less, still more preferably 3000 ppm by mass or less, and particularly preferably 2000 ppm by mass or less from the viewpoint of allowing the heat resistance of a resin molded body to be favorable.

The upper and lower limit values described above can be optionally combined. Preferred examples of the range of the content of the α-olefin with respect to the total mass of the resin composition include ranges of 0.1 ppm by mass or more and 10000 ppm by mass or less, 10 ppm by mass or more and 5000 ppm by mass or less, 60 ppm by mass or more and 4000 ppm by mass or less, 80 ppm by mass or more and 3000 ppm by mass or less, and 100 ppm by mass or more and 2000 ppm by mass or less. Of these, the content of the α-olefin is more preferably 10 ppm by mass or more and 5000 ppm by mass or less, and still more preferably 100 ppm by mass or more and 2000 ppm by mass or less.

The total content of the methyl propionate, the methyl pyruvate, and the methyl 2-methylbutyrate with respect to the total mass of the resin composition according to the present embodiment is typically 26 ppm by mass or more, preferably 30 ppm by mass or more, more preferably 40 ppm by mass or more, still more preferably 60 ppm by mass or more, particularly preferably 80 ppm by mass or more, and most preferably 100 ppm by mass or more from the viewpoint of obtaining excellent light stability.

The upper limit of the total content of the methyl propionate, the methyl pyruvate, and the methyl 2-methylbutyrate with respect to the total mass of the resin composition according to the present embodiment is not particularly limited, and is preferably 20000 ppm by mass or less, more preferably 15000 ppm by mass or less, still more preferably 10000 ppm by mass or less, particularly preferably 5000 ppm by mass or less, and most preferably 3000 ppm by mass or less from the viewpoint of allowing the heat resistance of a resin molded body to be favorable.

The upper and lower limit values described above can be optionally combined. Examples of the range of the total content of the methyl propionate, the methyl pyruvate, and the methyl 2-methylbutyrate include ranges of more than 26 ppm by mass and 20000 ppm by mass or less, 30 ppm by mass or more and 20000 ppm by mass or less, 40 ppm by mass or more and 15000 ppm by mass or less, 60 ppm by mass or more and 10000 ppm by mass or less, 80 ppm by mass or more and 5000 ppm by mass or less, and 100 ppm by mass or more and 3000 ppm by mass or less. Of these, the total content of the methyl propionate, the methyl pyruvate, and the methyl 2-methylbutyrate is more preferably 30 ppm by mass or more and 20000 ppm by mass or less, and still more preferably 40 ppm by mass or more and 15000 ppm by mass or less.

### <3-1. Operation-Effects>

The monomer composition according to the first embodiment of the present invention includes methyl methacrylate, an α-olefin, and a specific content of at least one compound selected from the group consisting of methyl propionate, methyl pyruvate, and methyl 2-methylbutyrate. A resin composition obtained by radical polymerization of a polymerizable composition (X2) including the monomer composition has excellent heat resistance and excellent light stability, and is inhibited from yellowing.

The reason why the monomer composition according to the first embodiment of the present invention includes a specific α-olefin and a specific content of at least one compound selected from the group consisting of methyl propionate, methyl pyruvate, and methyl 2-methylbutyrate to thereby obtain the resin composition that has excellent heat resistance and excellent light stability, and is inhibited from yellowing is presumed to be as follows.

In a polymer (methacrylic polymer) including a unit based on methyl methacrylate, the main chain or side chain is cleaved by light, to generate radical species. The generated radical species commonly results in yellowing of a methacrylic resin and a decrease in mechanical strength due to a decrease in molecular weight.

However, an α-olefin and a specific content of at least one compound selected from the group consisting of methyl propionate, methyl pyruvate, and methyl 2-methylbutyrate included in the monomer composition according to the embodiment of the first present invention do not provide a resonance stabilization effect and have considerably low reactivity in comparison with methyl methacrylate which is a conjugate monomer. Therefore, an unreacted α-olefin (also referred to as "α-olefin monomer") and at least one unreacted compound selected from the group consisting of methyl propionate, methyl pyruvate, and methyl 2-methylbutyrate (also referred to as "at least one compound monomer selected from the group consisting of methyl propionate, methyl pyruvate, and methyl 2-methylbutyrate") remain in the obtained resin composition unless under special conditions. The unreacted α-olefin and at least one compound selected from the group consisting of methyl propionate, methyl pyruvate, and methyl 2-methylbutyrate are considered to function as radical scavengers that scavenge the radical species. As a result, a hydrogen atom bound to a carbon atom adjacent to a double bond site is extracted from the unreacted α-olefin, scavenging the radical species.

At this time, it is considered that the at least one unreacted compound selected from the group consisting of the methyl propionate, the methyl pyruvate, and the methyl 2-methylbutyrate interacts with the unreacted α-olefin, from which the hydrogen atom has been extracted, to substitute the hydrogen atom, whereby the α-olefin functions as a radical scavenger again. Accordingly, it is considered that the light stability of the obtained resin composition exhibits considerably favorable light stability not only by the effect of improving light stability of the obtained resin composition by the α-olefin and at least one compound selected from the group consisting of the methyl propionate, the methyl pyruvate, and the methyl 2-methylbutyrate alone but also by the synergistic effect of combination of the α-olefin and at least one compound selected from the group consisting of the methyl propionate, the methyl pyruvate, and the methyl 2-methylbutyrate.

In the resin composition according to the present embodiment, the upper limit of the ratio of the content of α-olefin with respect to the total content of methyl propionate, methyl pyruvate, and methyl 2-methylbutyrate (also referred to as "ratio of [mass of α-olefin]/[total mass of methyl propionate, methyl pyruvate, and methyl 2-methylbutyrate]") is not particularly limited, and is preferably 1000 or less, more preferably 500 or less, still more preferably 300 or less, even more preferably 100 or less, and particularly preferably 10 or less from the viewpoint of allowing the light stability of the resin molded body to be favorable due to the interaction between at least one compound selected from the group consisting of the methyl propionate, the methyl pyruvate, and the methyl 2-methylbutyrate, and the α-olefin. The lower limit of the ratio of [mass of α-olefin]/[total mass of methyl propionate, methyl pyruvate, and methyl 2-methylbutyrate] is not particularly limited, and is preferably 0.00001 or more, more preferably 0.0001 or more, still more preferably 0.001 or more, even more preferably 0.01 or more, and particularly preferably 0.5 or more from the viewpoint of allowing the heat resistance of the resin molded body to be favorable.

The upper and lower limit values described above can be optionally combined. Preferred examples of the range of the ratio of [mass of α-olefin]/[total mass of methyl propionate, methyl pyruvate, and methyl 2-methylbutyrate] include ranges of 0.00001 or more and 1000 or less, 0.0001 or more and 500 or less, 0.001 or more and 300 or less, 0.01 or more and 100 or less, and 0.5 or more and 10 or less. Of these, the ratio of [mass of α-olefin]/[total mass of methyl propionate, methyl pyruvate, and methyl 2-methylbutyrate] is more preferably 0.0001 or more and 500 or less, and still more preferably 0.001 or more and 300 or less.

### <3-2. Methacrylic Polymer (P)>

The methacrylic polymer (P) is one of the components included in the resin composition according to the present embodiment.

The resin composition includes the methacrylic polymer (P), whereby transparency can be improved, decomposition due to heat or light is suppressed, and thermoformability, heat resistance, and mechanical strength can be allowed to be favorable. Furthermore, heat resistance inherent to the methacrylic polymer (P), and the synergistic effect between an α-olefin and a specific content of at least one compound selected from the group consisting of methyl propionate, methyl pyruvate, and methyl 2-methylbutyrate enable the obtainment of a methacrylic resin molded body by which the light stability of a resin composition obtained is high, and of which the heat resistance is maintained.

The content rate of a methyl methacrylate unit in the methacrylic polymer (P) is not particularly limited, and is preferably 50% by mass or more, more preferably 60% by mass or more, still more preferably 70% by mass or more, even more preferably 80% by mass or more, particularly preferably 90% by mass or more, and typically 100% by mass or less with respect to the total mass of the methacrylic polymer (P) from the viewpoint of allowing heat resistance to be favorable.

The methacrylic polymer (P) is preferably a copolymer methacrylic polymer (P1) including a methyl methacrylate unit and, if necessary, a repeating unit derived from an acrylic ester (hereinafter also referred to as "acrylic ester unit") or a styrene unit. The arrangement of these copolymers is not particularly limited, and may be, for example, a random copolymer, a block copolymer, or an alternating copolymer, but a random copolymer is preferred.

The repeating unit derived from the acrylic ester is a repeating unit derived from an acrylic ester having an alkyl group having 1 to 6 carbon atoms in the side chain. The monomer included in the unit is not particularly limited as long as the monomer is a monomer that can be copolymerized with methyl methacrylate. Examples thereof include acrylic esters such as methyl acrylate, ethyl acrylate, propyl acrylate, n-butyl acrylate, and tert-butyl acrylate. These may be used singly, or in optional combination of two or more kinds thereof at an optional ratio. Of these monomers, the monomer is preferably at least one acrylic ester selected from the group consisting of methyl acrylate, ethyl acrylate, and n-butyl acrylate, and more preferably methyl acrylate or ethyl acrylate, from the viewpoint of securing the high light stability of a resin molded body including the resin composition.

The content rate of the methyl methacrylate unit in the methacrylic polymer (P1) is not particularly limited, and is preferably 50% by mass or more, more preferably 60% by mass or more, still more preferably 70% by mass or more, still more preferably 80% by mass or more, particularly preferably 90% by mass or more, and typically 100% by mass or less with respect to the total mass of the methacrylic polymer (P1) from the viewpoint of allowing heat resistance to be favorable.

The content rate of the acrylic ester unit in the methacrylic polymer (P1) is not particularly limited, and is preferably 50% by mass or less, more preferably 30% by mass or less, still more preferably 20% by mass or less, particularly preferably 10% by mass or less, and typically 0% by mass or more from the viewpoint of allowing heat resistance and light stability to be favorable. In a case in which the methacrylic polymer (P1) includes two or more kinds of acrylic ester units, the content rate described above is the total content rate of the two or more kinds of acrylic ester units.

The content rate of the styrene unit in the methacrylic polymer (P1) is not particularly limited, and is preferably 50% by mass or less, more preferably 40% by mass or less, still more preferably 30% by mass or less, even more preferably 20% by mass or less, particularly preferably 10% by mass or less, and typically 0% by mass or more from the viewpoint of allowing transparency to be favorable.

Furthermore, the methacrylic polymer (P) in the present embodiment can include a structural unit derived from a polyfunctional monomer including two or more radical polymerizable functional groups in one molecule (hereinafter referred to as "polyfunctional monomer unit") within the range in which the effects of the invention can be obtained.

The radical polymerizable functional group referred to herein may be any group having a carbon-carbon double bond and capable of radical polymerization, and specific examples thereof include a vinyl group, an allyl group, a (meth)acryloyl group, and a (meth)acryloyloxy group. In particular, a (meth)acryloyl group is preferred from the viewpoint of the excellent storage stability of a compound having a radical polymerizable functional group and the ease of controlling the polymerizability of the compound. The radical polymerizable functional groups in a monomer having two radical polymerizable functional groups may be the same or different.

The methacrylic polymer (P) includes a polyfunctional monomer unit, whereby solvent resistance, chemical resistance, or the like can be improved.

Examples of the polyfunctional monomer include allyl methacrylate, allyl acrylate, ethylene glycol di(meth)acrylate, ethylene glycol tri(meth)acrylate, neopentyl glycol di(meth)acrylate, and trimethylolpropane tri(meth)acrylate, but are not particularly limited thereto. These may be used singly, or in optional combination of two or more kinds thereof at an optional ratio. Of these, the polyfunctional monomer is more preferably selected from ethylene glycol di(meth)acrylate and neopentyl glycol di(meth)acrylate, and still more preferably ethylene glycol di(meth)acrylate from the viewpoint of allowing solvent resistance and chemical resistance to be more favorable.

Furthermore, in the resin composition according to the present embodiment, the mass average molecular weight (Mw) of the methacrylic polymer (P), measured by gel permeation chromatography (GPC), is not particularly limited. The mass average molecular weight (Mw) can be set as appropriate depending on, for example, the application of the resin molded body. For example, the mass average molecular weight may be 10000 or more, 100000 or more, or 150000 or more, and may be 1000000 or less, 2000000 or less, or 4000000 or less. Solvent resistance and chemical resistance can be enhanced by increasing the mass average molecular weight as appropriate.

The mass average molecular weight (Mw) of the methacrylic polymer (P) can be controlled by adjusting polymerization temperature, polymerization time, the amount of added polymerization initiator, the kind of a series transfer agent, the amount of the added series transfer agent, or the like.

### <3-3. Methyl Isobutyrate>

The resin composition preferably further includes methyl isobutyrate. The inclusion of the compound enables a resin composition having further excellent light stability to be provided. This is presumed to further improve the effect of improving light stability due to the inclusion of an α-olefin and a certain amount of at least one compound selected from the group consisting of methyl propionate, methyl pyruvate, and methyl 2-methylbutyrate, described in <3-1. Operation-Effects>. Moreover, the deterioration of light stability in a case in which a resin molded body including the resin composition is exposed to light for a long time can be suppressed.

In a case in which the resin composition according to the present embodiment includes methyl isobutyrate, the total content of the methyl isobutyrate, methyl propionate, methyl pyruvate, and methyl 2-methylbutyrate with respect to the total mass of the resin composition is preferably an amount within the range of the total content of the methyl propionate, the methyl pyruvate, and the methyl 2-methylbutyrate, described above.

In a case in which the resin composition according to the present embodiment includes methyl isobutyrate, the content of the methyl isobutyrate with respect to the total mass of the resin composition is preferably 5 ppm by mass or more, more preferably 10 ppm by mass or more, still more preferably 15 ppm by mass or more, particularly preferably 30 ppm by mass or more, and most preferably 40 ppm by mass or more, and preferably 20000 ppm by mass or less, more preferably 5000 ppm by mass or less, still more preferably 1000 ppm by mass or less, particularly preferably 500 ppm by mass or more, and most preferably 100 ppm by mass or more.

The preferred upper and lower limit values described above can be optionally combined. Specifically, preferred examples of the range of the content of methyl isobutyrate with respect to the total mass of the resin composition according to the present embodiment include ranges of 5 ppm by mass or more and 20000 ppm by mass or less, 10 ppm by mass or more and 5000 ppm by mass or less, 15 ppm by mass or more and 1000 ppm by mass or less, 30 ppm by mass or more and 500 ppm by mass or less, and 40 ppm by mass or more and 100 ppm by mass or less.

### <3-4. Characteristics of Resin Composition>

The resin composition according to the present embodiment includes the methacrylic polymer (P), an α-olefin, and a specific amount of at least one compound selected from the group consisting of methyl propionate, methyl pyruvate, and methyl 2-methylbutyrate, and therefore has excellent light stability.

Specifically, when a test piece (square shape, 50 mm length × 50 mm width, 3 mm thickness) including a resin composition is subjected to a UV exposure test described below, the yellowness index (YI) of the test piece measured according to ASTM D1925 from before the start of the UV exposure test to 200 hours after the start of the UV exposure test is 6.2 or less, preferably 6.0 or less, more preferably 5.8 or less, still more preferably 5.5 or less, even more preferably 5.0 or less, and particularly preferably 4.8 or less.

### <4. Resin Molded Body>

A resin molded body according to a fourth embodiment of the present invention (also simply referred to as "resin molded body") is a resin molded body including the resin composition according to the third embodiment of the present invention. In other words, the resin molded body according to the present embodiment includes the resin composition according to the third embodiment of the present invention. A resin molded body having excellent light stability while maintaining transparency and heat resistance inherent to a methacrylic resin can be obtained through a molding step of molding the resin composition. Examples of a molding method in the molding step include press molding, injection molding, gas-assisted injection molding, weld molding, extrusion molding, blow molding, film molding, hollow molding, multilayer molding, and melt spinning. Herein, the resin molded body is not particularly limited as long as the resin molded body is a molded body including the resin composition described above, and a molded body consisting of only the resin composition substantially corresponds to both the resin composition and the resin molded body.

The shape of the resin molded body is not limited the following, but examples thereof include granular pellets, plate-shaped resin molded bodies (resin plates), and sheet- or film-shaped resin molded bodies (resin sheets). The thickness of the resin molded body can be adjusted to an optional thickness as needed, from a thick plate shape to a thin film shape. For example, thickness can be 0.1 µm or more and 30 mm or less, or 1 mm or more and 30 mm or less.

The resin molded body includes the resin composition described above, and is therefore excellent in light stability.

In other words, the resin molded body exhibits excellent light stability such that the yellowness index (YI), measured according to ASTM D1925, obtained between before the start of the UV exposure test described above and 200 hours after the start of the UV exposure test, is 6.0 or less, preferably 5.5 or less, more preferably 5.0 or less, still more preferably 4.5 or less, and particularly preferably 4.0 or less.

### <5. Method of Producing Resin Composition or Resin Molded Body>

A method of producing a resin composition or a resin molded body including the resin composition (hereinafter, the resin composition or the resin molded body is also generically referred to as "resin composition or the like") is not particularly limited. Specific examples of the method of producing the resin composition or the like include a method including a radical polymerization step of performing radical polymerization of the polymerizable composition (X2) according to the second embodiment of the present invention, preferably the polymerizable composition (X2) including the monomer composition according to the first embodiment of the present invention. The radical polymerization step may include a syrup preparation step of polymerizing a part of the polymerizable composition (X2) to prepare a syrup, and a polymerization step of polymerizing the polymerizable component in the syrup. In addition, "polymerizing a part of the polymerizable composition (X2)" in the syrup preparation step means polymerization such that the content of the methacrylic polymer in the obtained syrup is 10% by mass or more and 80% by mass or less, preferably 10% by mass or more and 60% by mass or less, more preferably 10% by mass or more and 40% by mass or less.

The polymerization temperature in the case of polymerizing the polymerizable composition (X2) is not particularly limited, and can be determined as appropriate by those skilled in the art according to well-known techniques. Typically, the temperature is set as appropriate in a range of preferably 40°C or more and 180°C or less, more preferably 50°C or more and 150°C or less, depending on the kind of a radical polymerization initiator used. In addition, the polymerizable composition (X2) can be polymerized under multistage temperature conditions as necessary. The polymerization time can be determined as appropriate depending on the progress of polymerization and curing.

Examples of the method of polymerizing the polymerizable composition (X2) include a bulk polymerization method, a suspension polymerization method, an emulsion polymerization method, and a dispersion polymerization method, among which a bulk polymerization method is preferred in view of productivity.

Specific examples of methods of producing a resin composition or the like include a method of obtaining a resin composition or the like by a bulk polymerization method using a known cast polymerization method such as a cell cast method or a continuous cast method, or a method of obtaining a resin composition or the like by molding a composition produced by a bulk polymerization method. It is more preferable to employ a method utilizing a cast polymerization (casting polymerization) method from the viewpoint of enabling further improvement in the heat resistance of the resin composition by increasing a molecular weight or introducing a crosslinked structure.

Examples of the cast polymerization method include a cell cast method in which in the case of obtaining a resin composition or the like having a plate shape, a space formed by two facing glass plates or metal plates (SUS plates) and a gasket such as a soft resin tube placed on the edge of the two plates is used as a mold, a syrup obtained by polymerizing the polymerizable composition (X2) or a part of the polymerizable composition (X2) is injected into the mold, the polymerization is completed by heat polymerization treatment, and the resin composition or the like is taken out from the mold. Alternatively, examples thereof include a continuous cast method in which a space formed by two stainless steel endless belts running in the same direction at the same speed and facing each other at a predetermined spacing and a gasket such as a soft resin tube placed on both sides of the belts is used as a mold, a syrup obtained by polymerizing the polymerizable composition (X2) or a part of the polymerizable composition (X2) is continuously injected from one end of the endless belt into the mold, the polymerization is completed by heat polymerization treatment, and the resin composition or the like is continuously taken out from the other end of the endless belt.

The spacing between gaps in the mold can be adjusted as appropriate by adjusting the thickness (diameter) of the gasket to obtain the resin composition or the like having a desired thickness. The thickness of the plate-shaped resin composition or the like is typically set in a range of 1 mm or more and 30 mm or less.

### <6. Applications>

The applications of the resin composition and resin molded body ("resin composition or the like") described above are not particularly limited. The resin composition and the resin molded body are used as members with light-transmitting properties, used in any of vehicular members, medical members, toys, liquid containers, optical materials, signboards, displays, decorative members, building members, and the face plates of electronic instruments, particularly preferably as transparent members.

### EXAMPLES

The features of the present invention are more specifically described below with reference to Examples and Reference Examples. The materials, amounts used, rates, treatment contents, treatment procedures, and the like, described in the following examples, can be changed as appropriate without departing from the gist of the present invention. Accordingly, the scope of the present invention should not be construed as being limited by the specific examples described below. In the following, the term "parts" denotes "parts by mass".

The abbreviations and names of compounds used in Examples and Reference Examples are as follows.
· Methyl methacrylate: Methyl methacrylate (manufactured by Mitsubishi Chemical Corporation)
·Methyl pyruvate (manufactured by Tokyo Chemical Industry Co., Ltd.)
· Methyl 2-methylbutyrate (manufactured by Tokyo Chemical Industry Co., Ltd.)
· Methyl propionate (manufactured by Tokyo Chemical Industry Co., Ltd.)
· 1-Octene (manufactured by Tokyo Chemical Industry Co., Ltd.) · 2-Ethyl-1-hexene (manufactured by Tokyo Chemical Industry Co., Ltd.)

Methyl methacrylate (manufactured by Mitsubishi Chemical Corporation) included 260 ppm by mass of methyl isobutyrate, 8 ppm by mass of methyl propionate, 19 ppm by mass of methyl pyruvate, and 8 ppm by mass of methyl 2-methylbutyrate with respect to the total mass of methyl methacrylate.

### [Measurement Method and Evaluation Method]

### <Method of Measuring Content of At Least One Compound Selected from Group Consisting of Transition Metal Compound and Group 13 Element Compound in Monomer Composition>

A monomer composition in 2 g was accurately weighed, and put in a Kjeldahl decomposition flask. Sulfuric acid in a volume of 3 mL was added, and the resultant was completely carbonized in a Kjeldahl decomposition apparatus, and then cooled. Sulfuric acid in a volume of 2 mL was added thereto, heated, and cooled, and 3 mL of nitric acid was put to perform decomposition again. This series of operations was repeated three times.

After the cooling, 3 mL of nitric acid and 1 mL of hydrogen peroxide were added, heated, and then cooled again, and this operation was repeated until the color disappeared. The nitric acid and hydrogen peroxide in the Kjeldahl flask were heated and volatilized, followed by adding sulfuric acid so that the content of sulfuric acid in the Kjeldahl flask became 5 mL. Thus, a solution was prepared. The total amount of this solution was transferred to a 100 mL measuring flask, and diluted with ultrapure water. The solution was subjected to quantification of each element under the following conditions using an ICP emission spectrophotometer.
Apparatus used: ICP emission spectrophotometer (manufactured by PerkinElmer, Inc., model name: Optima 8300)
Output: 1300 W
Pump speed: 1.0 mL/min
Plasma gas flow rate: 10 L/min
Auxiliary gas flow rate: 0.2 L/min
Nebulizer gas flow rate: 0.55 L/min
Detector: SCD (Segmented Array CCD)
Integral time: Auto (1 to 5 sec)
Number of measurements: 3
Measurement method: Absolute calibration curve method
Observation direction: Axial

### <Method of Measuring Amount of Target Substance Remaining in Resin Composition>

### (1) Procedure of Preparing Sample and Test Liquid

Each of the resin molded bodies obtained in Examples and Reference Examples was finely crushed, 0.2 g of the crushed resin was dissolved in 10 mL of acetone for residual pesticide testing (hereinafter simply referred to as "acetone"). After the dissolution of the resin, 1 mL of the internal standard solution was added with a whole pipette. A 0.1% by volume methyl salicylate/acetone solution was used as the internal standard solution. Three kinds of test liquids having different concentrations were prepared by diluting a target standard reagent with acetone, a three-point calibration curve was created by gas chromatography mass spectrometry (GC/MS) measurement described later, and the content of each target substance in the sample was quantified. A 0.1% by volume methyl salicylate/acetone solution was used as the internal standard solution.

### (GC/MS Measurement Conditions)

Apparatus: GC HP6890/MS HP5973 (manufactured by Agilent Technologies International Japan, Ltd.)
Ionization method: EI (electron ionization) method
Column: DB-WAX 60 m × 250 µm × 0.5 µm (manufactured by Agilent Technologies International Japan, Ltd.)
Temperature rising conditions: 70°C (5 min) → 200°C (5 min) Rate = 10°C/min Inlet temperature: 220°C
AUX temperature: 230°C
Ion source temperature: 230°C
Split ratio: 10: 1
Flow rate: 2.0 mL/min
Average linear velocity: 37 cm/sec
Injection amount: 1 µL
Measurement mode: SIM

### <Method of Evaluating Heat Resistance>

As an index of the heat resistance of the resin composition obtained in Examples and Reference Examples, a deflection temperature under load (hereinafter referred to as "HDT") (°C) was measured for the test pieces (length 127 mm × width 12.7 mm × thickness 3 mm) of the resin molded bodies obtained in Examples and Reference Examples in accordance with JIS K 7191.

### [Evaluation of Light Stability]

A change (ΔYI) in yellowness index was used as an index of the light stability of the resin composition produced in Examples and Reference Examples.

### <Light Stability Test>

A light stability test was conducted using a METAL WEATHER super-accelerated light stability test machine (model name: DW-R8PL-A, manufactured by Daypla Wintes Co., Ltd.) including a metal halide lamp (model: MW-60W, manufactured by Daypla Wintes Co., Ltd.) and a light cut filter (model: KF-1, manufactured Daypla Wintes Co., Ltd.). Specifically, a test piece (square shape, 50 mm length × 50 mm width, 3 mm thickness) including a resin composition was placed in the evaluation chamber of the METAL WEATHER super-accelerated light stability test machine, and the test piece was irradiated with light from a metal halide lamp for 300 hours. UV irradiation intensity was corrected so that the irradiation intensity at a wavelength of 300 to 400 nm measured with an ultraviolet (UV) illuminometer (model name: UVP-365-03, manufactured by Ushio Inc.) was 130 mW/cm². The test piece was irradiated with visible light and UV from a metal halide lamp. The interior of the evaluation chamber of the METAL WEATHER super-accelerated light stability test machine was set to be under an environment at a temperature of 63°C and a humidity of 50% RH.

### <Production of Resin Composition>

### [Example 1]

### (1) Production of Syrup

1-Octene as an α-olefin, and methyl propionate were added to a reactor (polymerization pot) including a cooling pipe, a thermometer, and a stirring machine, methyl methacrylate was further supplied, the resultant was bubbled with nitrogen gas while being stirred, and heating of the resultant was then started. When the internal temperature of the reactor reached 80°C, 0.12 parts of 2,2'-azobis(2,4-dimethylvaleronitrile) as a radical polymerization initiator and 0.075 parts of 1-dodecanethiol as a chain transfer agent were added and further heated until the internal temperature of the reactor reached 100°C. Then, the resultant was maintained for 9 minutes. Then, the resultant was cooled until the internal temperature of the reactor reached room temperature. Thus, a syrup containing 500 ppm of each of 1-octene and at least one compound selected from the group consisting of methyl propionate, methyl pyruvate, and methyl 2-methylbutyrate was obtained. The content of the polymer in the syrup was 25% by mass with respect to the total mass of the syrup.

### (2) Casting Polymerization

To 100 parts of the syrup described above, 0.15 parts of t-hexyl peroxypivalate as a radical polymerization initiator was added to obtain a polymerizable composition (X2). Then, the polymerizable composition (X2) was poured into a space at a gap spacing of 6.5 mm disposed by arranging a soft resin gasket at an SUS plate end between two facing SUS plates, and heated at 80°C for 30 minutes and then at 130°C for 30 minutes to cure the polymerizable composition (X2) to obtain a resin composition. The composition of the resin composition is set forth in Table 1. Then, the (meth)acrylic resin composition together with the SUS plate was cooled, and the SUS plate was then removed to obtain a plate-shaped resin molded body having a thickness of 5 mm. The evaluation results of the characteristics of the obtained resin molded body are set forth in Table 1. In Table 1, "-" means that no measurement was performed.

### [Examples 2 to 7, and Reference Examples 1 to 6]

Resin compositions and resin molded bodies were produced in a manner similar to that in Example 1, except that the composition of the monomer composition was changed as set forth in Tables 1 and 2. The compositions of the obtained resin compositions are set forth in Tables 1 and 2. The evaluation results of the characteristics of the obtained resin molded bodies are set forth in Tables 1 and 2.

In Examples 1 to 10, a monomer composition including methyl methacrylate, an α-olefin, and at least one compound selected from the group consisting of methyl propionate, methyl pyruvate, and methyl 2-methylbutyrate was used. In the monomer composition, the total content of methyl propionate, methyl pyruvate, and methyl 2-methylbutyrate with respect to the total mass of the monomer composition was more than 36 ppm by mass, and the α-olefin included at least one selected from the group consisting of 2-ethyl-1-hexene, 1-octene, and 1-dodecene. The resin compositions obtained by polymerizing these monomer compositions are resin compositions including a methacrylic polymer (P), an α-olefin, and at least one compound selected from the group consisting of methyl propionate, methyl pyruvate, and methyl 2-methylbutyrate. In the resin compositions, the total content of the methyl propionate, the methyl pyruvate, and the methyl 2-methylbutyrate was more than 26 ppm by mass, and the α-olefin included at least one selected from the group consisting of 2-ethyl-1-hexene, 1-octene, and 1-dodecene. It is found that the resin molded bodies obtained by molding the resin compositions have excellent light stability while maintaining transparency and heat resistance inherent to methacrylic resins.

On the other hand, the monomer compositions of Reference Examples 1, 2, 4, and 5 include α-olefins, but the total content of methyl propionate, methyl pyruvate, and methyl 2-methylbutyrate is 36 ppm by mass or less. The monomer composition of Reference Example 3 does not include α-olefins, and the total content of methyl propionate, methyl pyruvate, and the methyl 2-methylbutyrate is also 36 ppm by mass or less. The monomer compositions of Reference Examples 6 and 7 includes do not include α-olefins although the total content of methyl propionate, methyl pyruvate, and methyl 2-methylbutyrate is more than 36 ppm by mass. The resin compositions of Reference Examples 1, 2, 4, and 5 include α-olefins, but the total content of methyl propionate, methyl pyruvate, and methyl 2-methylbutyrate is 26 ppm by mass or less. The monomer composition of Reference Example 3 does not include α-olefins, and the total content of methyl propionate, methyl pyruvate, and methyl 2-methylbutyrate is also 26 ppm by mass or less. The monomer compositions of Reference Examples 6 and 7 include do not include α-olefins although the total content of methyl propionate, methyl pyruvate, and methyl 2-methylbutyrate is more than 36 ppm by mass.

When Examples 1 to 10 and Reference Example 3 are compared, it is found that the resin molded bodies obtained in Examples 1 to 10 maintain abilities equivalent to transparency and heat resistance inherent to resin molded bodies obtained by molding conventional methacrylic resins.

A common methacrylic resin molded body is required to have a transparency (YI at 0 hour after light irradiation) of 0.5 or less and a heat resistance (HDT) of 100°C or more. Therefore, it is found that the resin molded bodies obtained in Examples 1 to 10 have transparency and heat resistance exceeding the levels required for a common methacrylic resin molded body.

Furthermore, when Examples 1 to 10 and Reference Examples 1 to 7 are compared, it is found that the resin molded bodies obtained in Examples 1 to 10 have significantly higher light stability than the molded bodies obtained in Reference Examples 1 to 7.

Moreover, Examples 1 to 10 reveal that as long as the monomer composition and the resin composition include an α-olefin and not less than a specific amount of at least one compound selected from the group consisting of methyl propionate, methyl pyruvate, and methyl 2-methylbutyrate, a resin molded body excellent in transparency, heat resistance, and light stability can be obtained regardless of the content.

Furthermore, in Example 10, the monomer composition and the resin composition include an α-olefin and at least one compound selected from the group consisting of methyl propionate, methyl pyruvate, and methyl 2-methylbutyrate, and include two compounds selected from the group consisting of the methyl propionate, the methyl pyruvate, and the methyl 2-methylbutyrate. Example 10 reveals that when the total content of the methyl propionate, the methyl pyruvate, and the methyl 2-methylbutyrate is not less than a specific amount, a resin molded body exhibiting not only transparency and heat resistance equivalent to those of conventional methacrylic resin molded bodies but also significantly higher light stability can be obtained even if any compound is selected.

### [Table 1]

**Table1**

| | | | Example 1 | Example 2 | Example 3 | Example 4 | Example 5 | Example 6 | Example 7 | Example 8 | Example 9 | Example 10 |
|---|---|---|---|---|---|---|---|---|---|---|---|---|
| Monomer Composition | methyl methacrylate | parts by mass | 100 | 100 | 100 | 100 | 100 | 100 | 100 | 100 | 100 | 100 |
| | 1-octene | ppm by mass | 500 | 1000 | 50 | 150 | 50 | 300 | 500 | 750 | 150 | 50 |
| | 2-ethyl-1-hexene | ppm by mass | - | - | - | - | - | - | - | - | - | - |
| | methyl propionate | ppm by mass | 508 | 5008 | 8 | 8 | 8 | 8 | 308 | 8 | 8 | 33 |
| | methyl 2-methylbutyrate | ppm by mass | 8 | 8 | 58 | 158 | 8 | 8 | 8 | 8 | 8 | 33 |
| | methyl pyruvate | ppm by mass | 19 | 19 | 19 | 19 | 69 | 319 | 8 | 758 | 158 | 8 |
| | methyl isobutyrate | ppm by mass | 260 | 260 | 260 | 260 | 260 | 260 | 260 | 260 | 260 | 260 |
| | transition metal compound and/or Group 13 element compound | ppm by mass | 0 | 0 | 0 | 0 | 0 | 0 | 0 | 0 | 0 | 0 |
| Resin Composition | (meth)acrylic polymer (P) | mass % | 99.9 | 99.4 | 100.0 | 100.0 | 100.0 | 100.0 | 99.9 | 99.9 | 100.0 | 100.0 |
| | remaining content of 1-octene | ppm by mass | 460 | 830 | 50 | 134 | 43 | 267 | 465 | 647 | 116 | 40 |
| | remaining content of 2-ethyl-1-hexene | ppm by mass | - | - | - | - | - | - | - | - | - | - |
| | remaining content of methyl propionate | ppm by mass | 470 | 4600 | 2 | 2 | 2 | 2 | 271 | 2 | 2 | 25 |
| | remaining content of methyl 2-methylbutyrate | ppm by mass | 5 | 5 | 54 | 143 | 5 | 5 | 5 | 5 | 5 | 30 |
| | remaining content of methyl pyruvate | ppm by mass | 19 | 19 | 20 | 19 | 46 | 254 | 22 | 626 | 140 | 20 |
| | remaining content of methyl isobutyrate | ppm by mass | 49 | 49 | 51 | 50 | 50 | 49 | 50 | 49 | 49 | 50 |
| Evaluation Result of Resin molded body | YI | 0 h | 0.34 | 0.19 | 0.17 | 0.23 | 0.22 | 0.21 | 0.28 | 0.21 | 0.16 | 0.26 |
| | | 200 h | 5.98 | 4.72 | 5.30 | 5.44 | 5.35 | 4.50 | 5.14 | 4.83 | 5.79 | 5.75 |
| | weather resistance(ΔYI) | | 5.64 | 4.53 | 5.13 | 5.21 | 5.13 | 4.29 | 4.86 | 4.62 | 5.63 | 5.49 |
| | heat resistance (HDT) | °C | 102.6 | 101.4 | 103.6 | 103.4 | 104.1 | 103.9 | 103.0 | 102.3 | 101.8 | 103.2 |

### [Table 2]

**Table2**

| | | | Reference Example 1 | Reference Example 2 | Reference Example 3 | Reference Example 4 | Reference Example 5 | Reference Example 6 | Reference Example 7 |
|---|---|---|---|---|---|---|---|---|---|
| Monomer Composition | methyl methacrylate | parts by mass | 100 | 100 | 100 | 100 | 100 | 100 | 100 |
| | 1-octene | ppm by mass | 1500 | - | - | 100 | 300 | - | - |
| | 2-ethyl-1-hexene | ppm by mass | - | 1500 | - | - | - | - | - |
| | methyl propionate | ppm by mass | 8 | 8 | 8 | 8 | 8 | 308 | 8 |
| | methyl 2-methylbutyrate | ppm by mass | 8 | 8 | 8 | 8 | 8 | 8 | 8 |
| | methyl pyruvate | ppm by mass | 19 | 19 | 19 | 19 | 19 | 19 | 319 |
| | methyl isobutyrate | ppm by mass | 260 | 260 | 260 | 260 | 260 | 260 | 260 |
| | transition metal compound and/or Group 13 element compound | ppm by mass | 0 | 0 | 0 | 0 | 0 | 0 | 0 |
| Resin Composition | (meth)acrylic polymer (P) | mass % | 99.9 | 99.9 | 100.0 | 100.0 | 100.0 | 100.0 | 100.0 |
| | remaining content of 1-octene | ppm by mass | 1260 | - | - | 80 | 260 | - | - |
| | remaining content of 2-ethyl-1-hexene | ppm by mass | - | 1300 | - | - | - | - | - |
| | remaining content of methyl propionate | ppm by mass | 2 | 2 | 2 | 2 | 2 | 280 | 2 |
| | remaining content of methyl 2-methylbutyrate | ppm by mass | 5 | 5 | 5 | 5 | 5 | 5 | 5 |
| | remaining content of methyl pyruvate | ppm by mass | 19 | 19 | 19 | 19 | 19 | 19 | 280 |
| | remaining content of methyl isobutyrate | ppm by mass | 49 | 49 | 49 | 49 | 49 | 49 | 49 |
| Evaluation Result of Resin molded body | YI | 0 h | 0.25 | 0.22 | 0.28 | 0.28 | 0.25 | 0.38 | 0.33 |
| | | 200 h | 7.16 | 6.91 | 12.31 | 6.47 | 6.37 | 6.42 | 7.07 |
| | weather resistance(ΔYI) | | 6.91 | 6.69 | 12.03 | 6.19 | 6.12 | 6.04 | 6.74 |
| | heat resistance (HDT) | °C | 105.0 | 104.0 | 105.1 | 105.1 | 105.1 | 103.9 | 103.2 |

## Claims

1. A monomer composition comprising methyl methacrylate, an α-olefin, and at least one compound selected from the group consisting of methyl propionate, methyl pyruvate, and methyl 2-methylbutyrate, wherein
a total content of the methyl propionate, the methyl pyruvate, and the methyl 2-methylbutyrate is more than 36 ppm by mass with respect to a total mass of the monomer composition, and
the α-olefin comprises at least one selected from the group consisting of 2-ethyl-1-hexene, 1-octene, and 1-dodecene.

2. The monomer composition according to claim 1, wherein a content of the methyl methacrylate is 85% by mass or more with respect to the total mass of the monomer composition.

3. The monomer composition according to claim 1 or 2, wherein a content of the methyl methacrylate is 90% by mass or more with respect to the total mass of the monomer composition.

4. The monomer composition according to any one of claims 1 to 3, wherein a content of the α-olefin is 0.1 ppm by mass or more with respect to the total mass of the monomer composition.

5. The monomer composition according to any one of claims 1 to 4, wherein a content of the α-olefin is 10 ppm by mass or more with respect to the total mass of the monomer composition.

6. The monomer composition according to any one of claims 1 to 5, wherein a content of the α-olefin is 60 ppm by mass or more with respect to the total mass of the monomer composition.

7. The monomer composition according to any one of claims 1 to 6, wherein a content of the α-olefin is 80 ppm by mass or more with respect to the total mass of the monomer composition.

8. The monomer composition according to any one of claims 1 to 7, wherein a content of at least one compound selected from the group consisting of a transition metal compound and a Group 13 element compound is 2 × 10⁴ ppm by mass or less with respect to the total mass of the α-olefin.

9. The monomer composition according to any one of claims 1 to 8, wherein the total content of the methyl propionate, the methyl pyruvate, and the methyl 2-methylbutyrate is 40 ppm by mass or more with respect to the total mass of the monomer composition.

10. The monomer composition according to any one of claims 1 to 9, wherein the total content of the methyl propionate, the methyl pyruvate, and the methyl 2-methylbutyrate is 60 ppm by mass or more with respect to the total mass of the monomer composition.

11. The monomer composition according to any one of claims 1 to 10, wherein the total content of the methyl propionate, the methyl pyruvate, and the methyl 2-methylbutyrate is 70 ppm by mass or more with respect to the total mass of the monomer composition.

12. The monomer composition according to any one of claims 1 to 11, wherein the total content of the methyl propionate, the methyl pyruvate, and the methyl 2-methylbutyrate is 100 ppm by mass or more with respect to the total mass of the monomer composition.

13. The monomer composition according to any one of claims 1 to 12, wherein a ratio of a content of the α-olefin to the total content of the methyl propionate, the methyl pyruvate, and the methyl 2-methylbutyrate ([a mass of the α-olefin]/[a total mass of the methyl propionate, the methyl pyruvate, and the methyl 2-methylbutyrate]) is 0.00001 or more and 1000 or less.

14. The monomer composition according to any one of claims 1 to 13, further comprising an acrylic ester.

15. The monomer composition according to claim 14, wherein the acrylic ester is at least one compound selected from the group consisting of methyl acrylate, ethyl acrylate, and n-butyl acrylate.

16. The monomer composition according to claim 14, wherein the acrylic ester is methyl acrylate or ethyl acrylate.

17. The monomer composition according to any one of claims 1 to 16, further comprising styrene.

18. The monomer composition according to any one of claims 1 to 17, further comprising methyl isobutyrate.

19. A method of producing a resin composition, the method comprising a radical polymerization step of performing radical polymerization of a polymerizable composition comprising the monomer composition according to any one of claims 1 to 18.

20. A resin composition comprising a polymer of the monomer composition according to any one of claims 1 to 18.

21. A resin composition comprising a methacrylic polymer (P), an α-olefin, and at least one compound selected from the group consisting of methyl propionate, methyl pyruvate, and methyl 2-methylbutyrate, wherein
a total content of the methyl propionate, the methyl pyruvate, and the methyl 2-methylbutyrate is more than 26 ppm by mass with respect to a total mass of the resin composition, and
the α-olefin comprises at least one selected from the group consisting of 2-ethyl-1-hexene, 1-octene, and 1-dodecene.

22. The resin composition according to claim 21, wherein the methacrylic polymer (P) comprises 50% by mass or more of a repeating unit derived from methyl methacrylate with respect to a total mass of the methacrylic polymer (P).

23. The resin composition according to claim 21 or 22, wherein the methacrylic polymer (P) comprises 70% by mass or more of a repeating unit derived from methyl methacrylate with respect to a total mass of the methacrylic polymer (P).

24. The resin composition according to any one of claims 21 to 23, wherein a content of the α-olefin is 0.1 ppm by mass or more with respect to the total mass of the resin composition.

25. The resin composition according to any one of claims 21 to 24, wherein a content of the α-olefin is 10 ppm by mass or more with respect to the total mass of the resin composition.

26. The resin composition according to any one of claims 21 to 25, wherein a content of the α-olefin is 60 ppm by mass or more with respect to the total mass of the resin composition.

27. The resin composition according to any one of claims 21 to 26, wherein a content of the α-olefin is 80 ppm by mass or more with respect to the total mass of the resin composition.

28. The resin composition according to any one of claims 21 to 27, wherein the total content of the methyl propionate, the methyl pyruvate, and the methyl 2-methylbutyrate is 30 ppm by mass or more with respect to the total mass of the resin composition.

29. The resin composition according to any one of claims 21 to 28, wherein the total content of the methyl propionate, the methyl pyruvate, and the methyl 2-methylbutyrate is 40 ppm by mass or more with respect to the total mass of the resin composition.

30. The resin composition according to any one of claims 21 to 29, wherein the total content of the methyl propionate, the methyl pyruvate, and the methyl 2-methylbutyrate is 60 ppm by mass or more with respect to the total mass of the resin composition.

31. The resin composition according to any one of claims 21 to 30, wherein the total content of the methyl propionate, the methyl pyruvate, and the methyl 2-methylbutyrate is 80 ppm by mass or more with respect to the total mass of the resin composition.

32. The resin composition according to any one of claims 21 to 31, further comprising methyl isobutyrate in the resin composition.

33. The resin composition according to any one of claims 21 to 32, wherein the methacrylic polymer (P) comprises a repeating unit derived from methyl methacrylate and a repeating unit derived from an acrylic ester.

34. The resin composition according to any one of claims 21 to 32, wherein the methacrylic polymer (P) comprises a repeating unit derived from methyl methacrylate and a repeating unit derived from styrene.

35. A resin molded body comprising the resin composition according to any one of claims 20 to 34.

36. A vehicular member comprising the resin molded body according to claim 35.

37. A medical member comprising the resin molded body according to claim 35.

38. A toy comprising the resin molded body according to claim 35.

39. A liquid container comprising the resin molded body according to claim 35.

40. An optical material comprising the resin molded body according to claim 35.

41. A signboard comprising the resin molded body according to claim 35.

42. A display comprising the resin molded body according to claim 35.

43. A method of producing a resin molded body, wherein
the method comprises a molding step of molding a resin composition comprising a methacrylic polymer (P), an α-olefin, and at least one compound selected from the group consisting of methyl propionate, methyl pyruvate, and methyl 2-methylbutyrate,
a total content of the methyl propionate, the methyl pyruvate, and the methyl 2-methylbutyrate in the resin composition is more than 26 ppm by mass with respect to a total mass of the resin composition, and
the α-olefin comprises at least one selected from the group consisting of 2-ethyl-1-hexene, 1-octene, and 1-dodecene.
